# EUROPEAN PATENT SPECIFICATION

(11) **EP 0 718 285 B1**
(45) Date of publication and mention of the grant of the patent: **11.04.2001**
(21) Application number: 95309171.7
(22) Date of filing: 18.12.1995
(51) Int. Cl.: C07C 403/20, C07C 69/618, C07C 57/50, C07D 277/34, C07D 307/87, A61K 31/23, A61K 31/20

(54) **Restricted 9-cis-retinoids**
Beschränkte 9-cis-Retinoide
9-cis-Rétinoides restreints

(30) Priority: 19.12.1994 US 359141; 14.11.1995 US 542146
(43) Date of publication of application: 26.06.1996
(60) Divisional application: 00101311.9
(73) Proprietor: American Cyanamid Company, Madison, New Jersey 07940-0874 (US)
(72) Inventor: Quing, Feng-Ling, Shanghai 200032 (CN); Birnberg, Gary H., Monroe, New York 10950 (US); Epstein, Joseph W., Monroe, New York 10950 (US); Gilbert, Adam M., Valley Cottage, New York 10989 (US)
(74) Representative: Wileman, David Francis, Dr.

(56) References cited:
- US-A- 5 219 888
- EUROPEAN JOURNAL OF MEDICINAL CHEMISTRY CHIMICA THERAPEUTICA., vol. 15, no. 1, 1980, PARIS FR, pages 9-15, XP002004638 P. LOELIGER ET AL.: "Arotinoids, a new class of highly active retinoids"

## Description

This invention relates to cis-retinoids, more particularly to restricted 9-cis-retinoids useful in the treatment of coronary artery disease, processes for preparing them and pharmaceutical compositions containing them.

### Description of the Prior Art

The family of molecules comprising both the natural and synthetic analogs of retinol (Vitamin A), are potent agents for control of both cellular differentiation and cellular proliferation (Wolbach et al, J. Exp. Med., 42:753-777). High Density Lipoproteins (HDL), a heterogeneous population of spherical particles containing variable amounts of lipids and apolipoprotein, are the most abundant lipoproteins in the plasma. It has recently been observed that low plasma HDL levels are associated with an increased incidence of coronary artery disease (CAD). Numerous epidemiological studies over the last thirty years have verified this association and provided evidence for a putative protective effect of increased HDL levels against CAD (Miller, N. E. , Am Heart J, 113:589-597 (1987). It is believed that HDL plays a fundamental role in the lipid transport system and that HDL represents a site for transport storage of potentially harmful lipids and apolipoproteins which, if they were not packaged into lipoprotein particles, might damage cell membranes because of their potential detergent properties (Eisenberg, S., J. Lipid Res. 25:1017-1058(1984)).

It is known that high-density lipoproteins are involved in a large number of diverse intravascular metabolic processes including the process of reverse cholesterol transport, in which cholesterol from extrahepatic tissue is transported to the liver for conversion to bile acids and eventual excretion. As a result of the observations, research efforts have focused on methods of affecting plasma HDL levels in order to provide protection against CAD.

As stated above, spherical particles of HDL contain variable amounts of lipoproteins and apolipoproteins. Apolipoprotein A-I (Apo A-I) is a major protein constituent of plasma HDL and intestinally derived lipoproteins known as chylomicrons. Although recent studies suggest that dietary, hormonal and other environmental factors regulate Apo A-I gene expression, the molecular basis for the mechanisms involved remains poorly understood. It is known that the gene coding for apolipoprotein A-I is expressed predominantly in the liver and intestine. Previous work has shown that hepatocyte-specific expression is determined by synergistic interactions between transcription factors bound to three separate sites with a powerful liver-specific enhancer located on the -222 to -110 nucleotides upstream of the apolipoprotein A-I start site (Widom et al, Mol. Cell Biol., 11:677-678 (1991)). In a recent study, it was found that one of the sites in this enhancer is a highly specific retinoic acid-responsive element, RARE, that responds to recently identified retinoic acid receptors, RXRα, (Rottman et al, Mol. Cell Biol., 11:3814-3820 (July 1991). These results suggest that retinoic aid response pathways mediated by RXRα play a role in apolipotrotein A-I expression and ultimately cholesterol and retinoid transport and metabolism.

Ringer el al, Am. J. Chem. Nutr., 53:688-694 (1991) observed an increase in HDL concentrations in patients given β-carotene, but did not find any changes in apolipoprotein A or B levels. Gollinich et al, Saurat (ed.), Retinoids: New Finds in Research and Therapy, Retinoid Symp., Geneva 1984, pp 445-460 (Karger, Basel 1985) reported no significant alteration in the HDL and LDL fractions of cholesterol in patients given etretinoate, and a decrease in HDL-chlolesterol under isotretinoin. Lyons et al, Br. J. Dermotology, 107:591-595 (1982) observed a decrease in HDL-cholesterol levels in patients given 13-cis-retinoic acid.

### SUMMARY OF THE INVENTION

The invention is novel analogs of 9-cis-retinoic acid which are useful for the treatment and prevention of coronary artery disease and to protect against premature atherosclerosis by increasing HDL levels. Additionally, compounds of this invention are useful in the treatment of cancers by the induction of tumor cell differentiation. The invention includes processes for preparing the novel 9-cis-retinoic acid analogs.

Compounds of the invention are represented by Formula I: 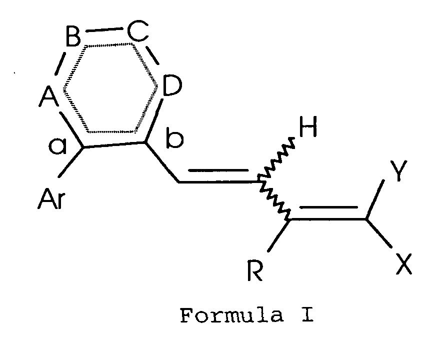 wherein:
A, B and C are CH, CH₂, O, or S wherein no more than one heteroatom is present;
D is (CH)ₘ, and m is an integer 0 to 1; or D is (CH₂)ₙ, and n is an integer 0 to 2;
the dotted line ,----, represents the presence or absence of a double bond whereby if only one double bond is present, it is disposed to the a-b position; or if multiple double bonds are present they are in a conjugated position to produce an aromatic ring;
R is hydrogen, methyl, ethyl, t-butyl, or trifluoromethyl;
Ar is:
   a moiety of the formula: 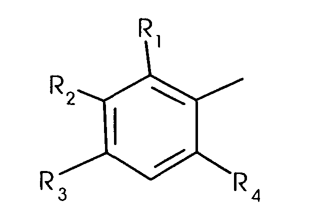
wherein R₁, R₂, R₃, and R₄ are hydrogen, (C₁-C₃) alkyl, (C₁-C₃)alkoxy or trifluoromethyl;
   or a moiety of the formula: 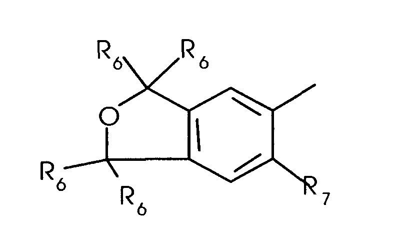
wherein R₆ is hydrogen, methyl or ethyl; and
R₇ is hydrogen, methyl or ethyl; Y is hydrogen, and
X is CH₂OH; CHO; CO₂H; CN; CH₂CONH₂ or a moiety of the formula: 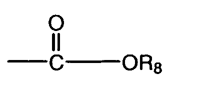
wherein R₈ is straight or branched (C₁-C₈)alkyl, or 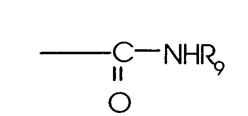
wherein R₉ is hydrogen, straight or branched (C₁-C₁₀) alkyl, glycosyl, 2-methoxyethyl, 2-dimethylaminoethyl, (2,3 or 4)-pyridyl, or (2,3 or 4)-pyridylmethyl; or X and Y taken together form the thiazolidinedione ring of the formula: 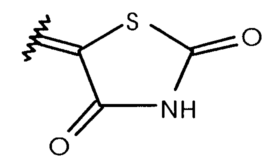 and the pharmaceutically acceptable salts and esters.

A preferred embodiment of compounds of Formula I is: 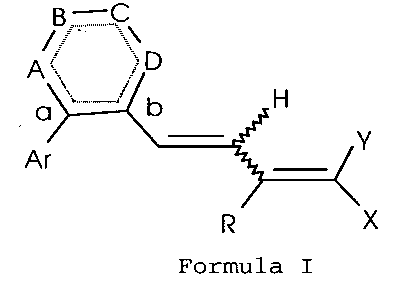 wherein:
A, B and C are CH, CH₂, O, or S wherein no more than one heteroatom is present;
D is (CH)ₘ, and m is an integer 0 to 1; or D is (CH₂)ₙ, and n is an integer 0 to 2;
the dotted line, -----, represents the presence or absence of a double bond whereby if only one double bond is present, it is disposed to the a-b position; or if multiple double bonds are present they are in a conjugated position to produce an aromatic ring;
R is hydrogen, methyl, ethyl, t-butyl, or trifluoromethyl;
Ar is:
   a moiety of the formula: 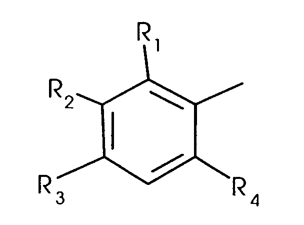
wherein R₁, R₂, R₃, and R₄ are hydrogen, (C₁-C₃)alkyl, (C₁-C₃)alkoxy or trifluoromethyl; Y is hydrogen, and
X is CH₂OH, CHO, CO₂H, CN, CH₂CONH₂, or a moiety of the formula: 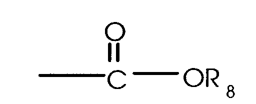
and R₈ is straight or branched (C₁-C₈)alkyl, or 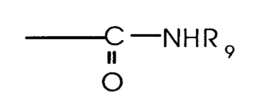
wherein R₉ is hydrogen, straight or branched (C₁-C₁₀)alkyl, glycosyl, 2-methoxyethyl, 2-dimethylaminoethyl, (2,3 or 4)-pyridyl, or (2,3 or 4)-pyridylmethyl; or X and Y taken together form the thiazolidinedione ring of the formula: 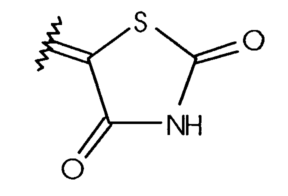 and the pharmaceutically acceptable salts and esters.

Further preferred embodiments of Formula I are: 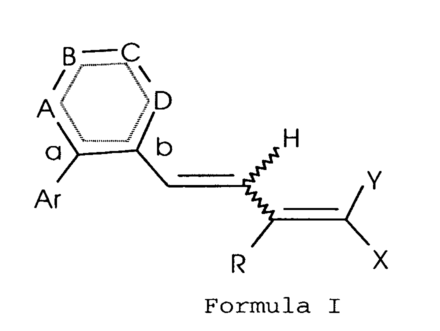 wherein:
A, B and C are CH, CH₂, O, or S wherein no more than one heteroatom is present;
Y is hydrogen;
D is (CH)ₘ, and m is an integer 0 to 1; or D is (CH₂)ₙ, and n is an integer 0 to 2;
the dotted line, -----, represents the presence or absence of a double bond whereby if only one double bond is present, it is disposed to the a-b position;
or if multiple double bonds are present they are in a conjugated position to produce an aromatic ring;
R is hydrogen, methyl, ethyl, t-butyl, or trifluoromethyl;
Ar is:
   a moiety of the formula: 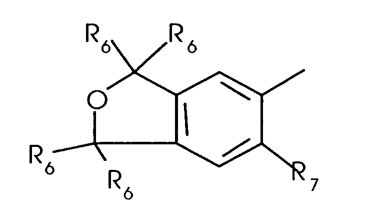
wherein R₆ is hydrogen, methyl or ethyl; and
R₇ is hydrogen, methyl or ethyl; Y is hydrogen, and
X is CH₂OH, CHO, CO₂H, CN, CH₂CONH₂, or a moiety of the formula: 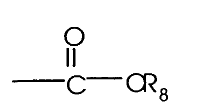
and R₈ is (C₁-C₈) alkyl, or 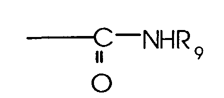
wherein R₉ is hydrogen, straight or branched (C₁-C₁₀) alkyl, glycosyl, 2-methoxyethyl, 2-dimethylaminoethyl, (2,3 or 4)-pyridyl, or (2,3 or 4)-pyridylmethyl; or X and Y taken together form the thiazolidinedione ring of the formula: 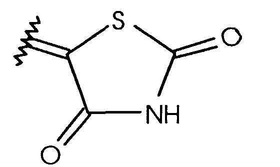 and the pharmaceutically acceptable salts and esters.

### BRIEF DESCRIPTION OF THE FIGURES

- Figure 1: shows the effect of retinoid analogs and trans-retinoic acid on HDL cholesterol.
- Figure 2: shows the effect of retinoid analogs and trans-retinoic acid on plasma Apo A-I

This invention also provides processes for preparing the compounds of formula I comprising one of the following:
a)reacting a compound of formula 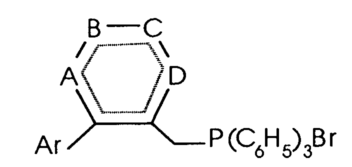 with an aldehyde of formula 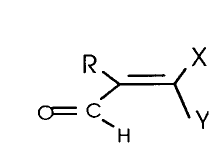
in which formulae Ar, A, B, C, D, R, and the dotted line are as defined above, X is CO₂R₈ and Y is hydrogen to give a corresponding compound of formula I;
   or
b) hydrolysing a compound of formula I where X ic COR₈ to give a carboxylic acid wherein X is CO₂H;
   or
c) reducing a compound of formula I where X is CO₂R₈ or CO₂H to give a compound of formula I wherein X is CH₂OH;
   or
d) reacting a compound of formula I wherein X is CO₂H with an activating reagent and followed by an amine of formula R₉NH₂ to give an amide of formula I wherein X is CONHR₉;
   or
e) reacting a compound of formula VIII 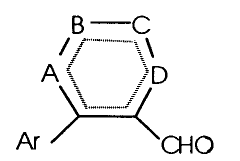 with a ylide of formula 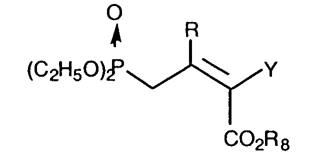 to give a corresponding compound of formula I wherein Ar, A, B, C, D, R, Y and R₈ are as defined above;
   or
f) oxidising a compound of formula I wherein X is CH₂OH to give a compound of formula I wherein X is CHO;
   or
g) dehydrating a compound of formula I wherein X is CONH₂ to give a compound of formula I wherein X is CN, eg using P₂O₅;
   or
h) reacting a compound of formula I wherein X is CN with sodium azide to gie a compound of formula I wherein X is 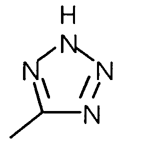 or
i) reacting an aldehyde of formula 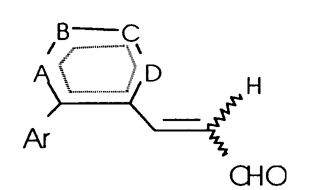 wherein Ar, A, B, C, D and the dotted line are as defined above with 2,4-thiazolidinedione to give a compound of formula I wherein X and Y taken together represent

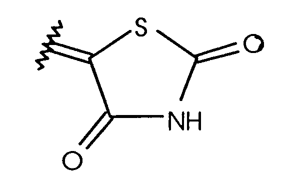

Starting materials used in the processes of the invention are known compounds or can be made by analogous methods for known compounds *(eg. vide infra).*

### DESCRIPTION OF THE PREFERRED EMBODIMENTS

According to Scheme 1, 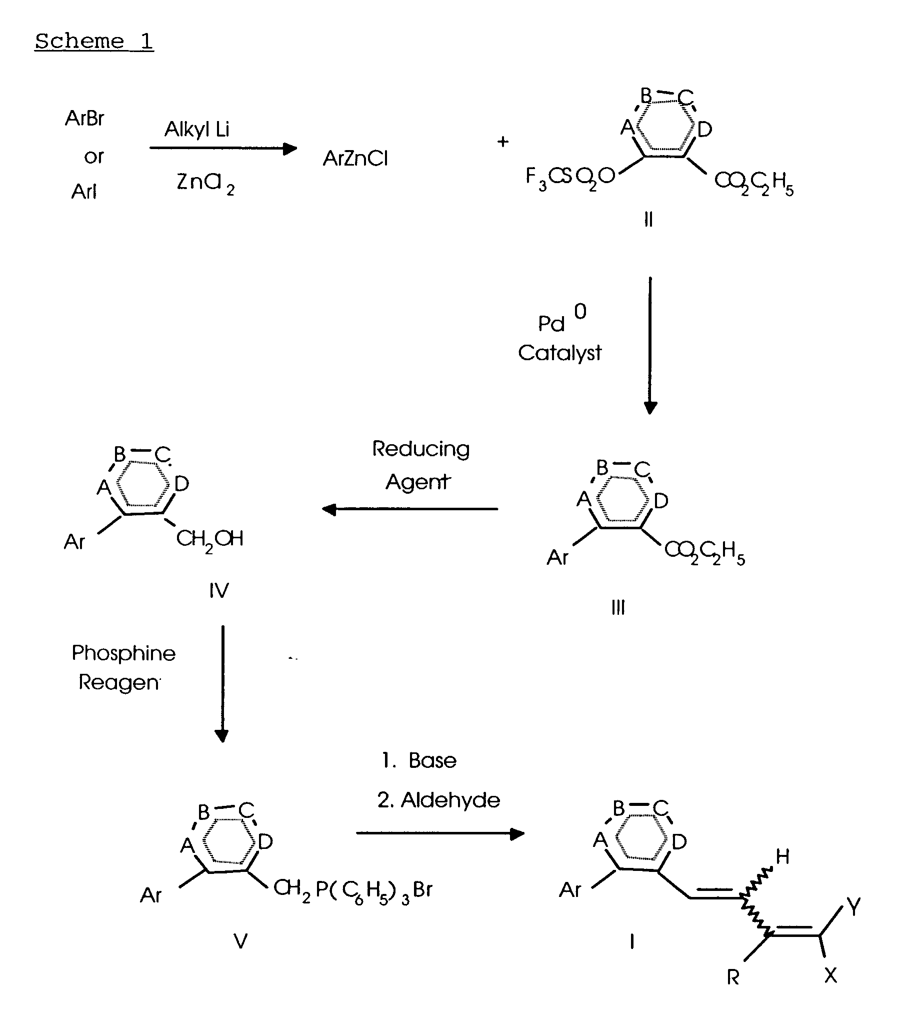 a compound of formula ArBr or ArI, wherein Ar is as defined hereinabove; is reacted with an alkyllithium such as tert-butyllithium, in an inert solvent such as tetrahydrofuran, at a temperature of -78°C to 30°C, for 1 to 5 hours, followed by ZnCl₂; to give a compound of the formula:

ArZnCl

which is in turn reacted with a compound of the formula II: 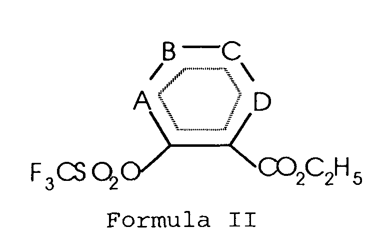 wherein A, B, C, D and the dotted line (---) are as defined hereinabove; in the presence of a palladium⁰ catalyst such as Pd[P(C₆H₅)₃]₄; in an inert solvent such as tetrahydrofuran; at 10 to 60°C for 1 to 5 hours; to give a compound of Formula III:

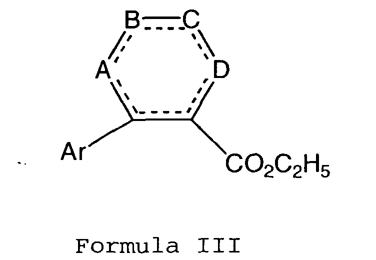

A compound of Formula III, wherein Ar, A, B, C, D and the dotted line (---) are as defined hereinabove; is reduced with a hydride reducing agent such as lithium aluminum hydride; in an inert solvent such as diethyl ether or tetrahydrofuran; at 0 to 60°C for 0.5 to 6.0 hours; to give a compound of Formula IV:

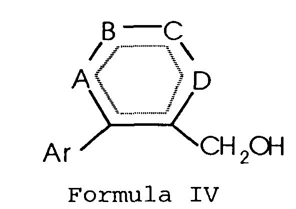

A compound of Formula IV, wherein Ar, A, B, C, D and the dotted line (---) are as defined hereinabove; is reacted with a phosphine such as triphenylphosphine hydrobromide; to give a compound of Formula V:

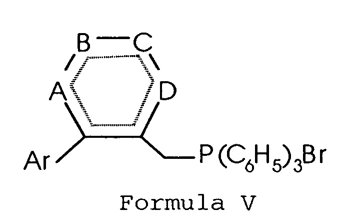

A compound of formula V is reacted with a base such as potassium hydroxide, sodium methoxide or sodium ethoxide; in a solvent such as methyl alcohol, ethyl alcohol or tetrahydrofuran; at 0°C for 0.5 to 3.0 hours followed by the addition of an aldehyde such as 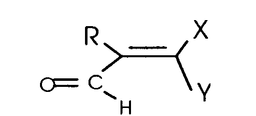 wherein R is as defined hereinabove; X is CO₂R₈ and R₈ is as defined hereinabove, y is hydrogen; to give a compound of Formula I.

According to Scheme 2, a compound of Formula I 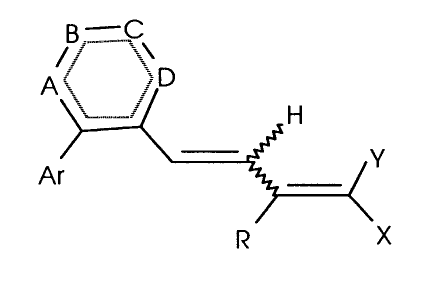 wherein Ar, A, B, C, D, R, Y, and the dotted line (---) are as defined hereinabove; X is -CO₂H or -CO₂R₈ and R8 is as defined hereinabove; is reacted with a hydride reducing agent such as lithium aluminum hydride; in an inert solvent such as tetrahydrofuran; at 0 to 60°C for 0.5 to 3.0 hours; to give a compound of Formula I wherein X is - CH₂OH. The resulting compound is then reacted with an oxidizing agent such as manganese dioxide to give a compound of Formula I wherein X is -CHO, and Y is hydrogen.

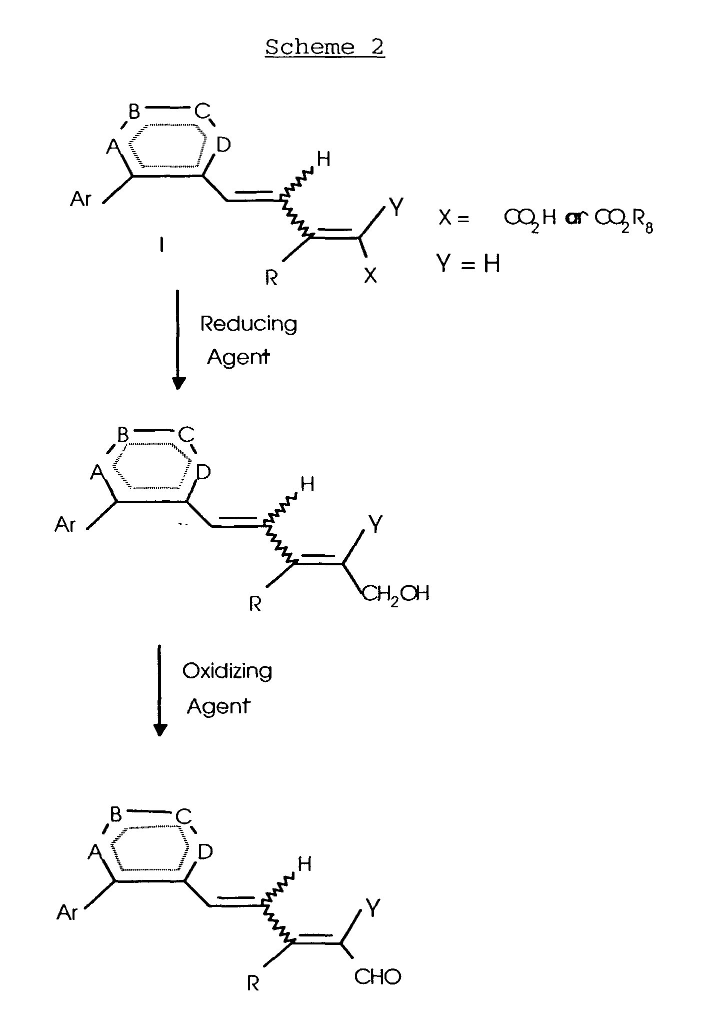

Alternatively, as shown in Scheme 3, a compound of Formula I, wherein Y is hydrogen; X is -CO₂R₈ and R₈ is as defined hereinabove, is reacted under hydrolysis conditions with a base such as sodium hydroxide or potassium hydroxide in water; at 30 to 100°C for 0.5 to 8 hours; followed by acidification with a mineral acid such as hydrochloric acid; to give a compound of Formula I wherein X is -CO₂H.

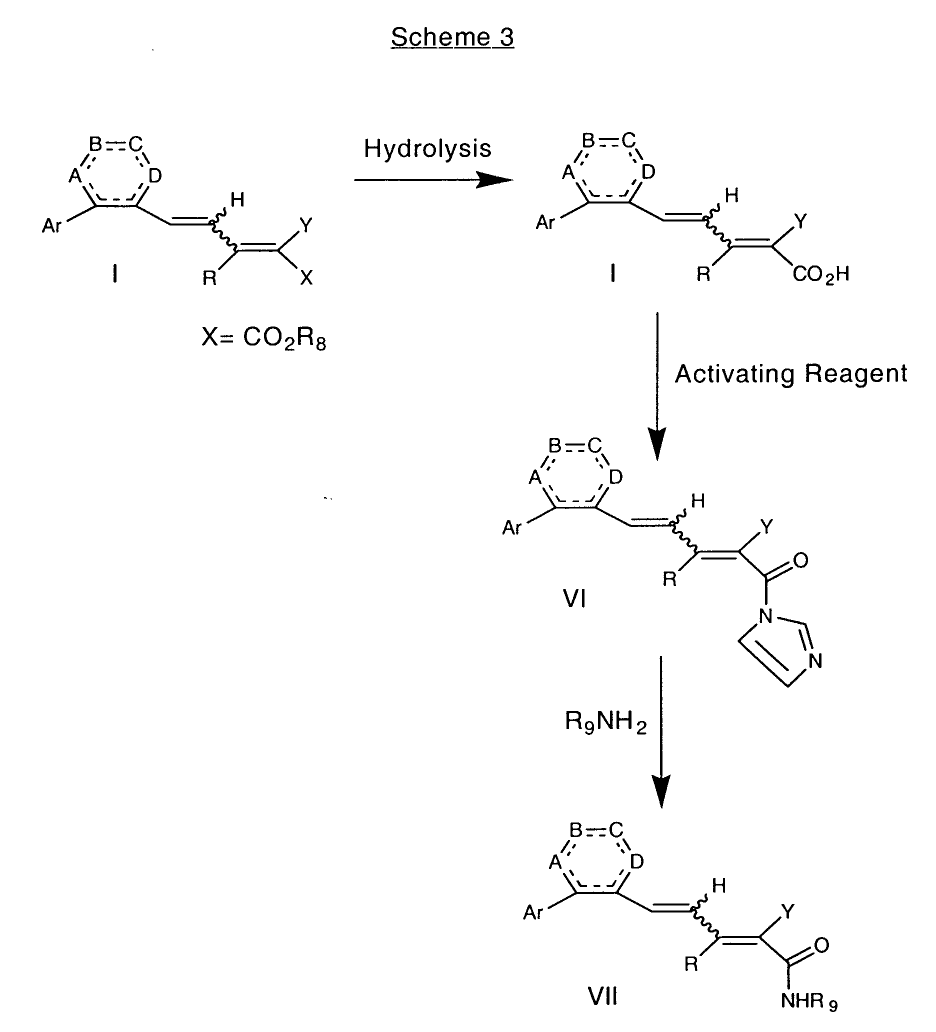

A compound of Formula I wherein Ar, A, B, C, D, the dotted line (---) and R and Y are as defined hereinabove and X is -CO₂H, is reacted with an activating reagent selected from carbonyldiimidazole, thionyl chloride, t-butyl-chloroformate, PCl₃, POCl₃, and PCl₅; in a solvent such as tetrahydrofuran; at 0 to 25°C for 0.5 to 1 hour; to give an intermediate of the Formula VI when the activating reagent is carbonyl-diimidazole: 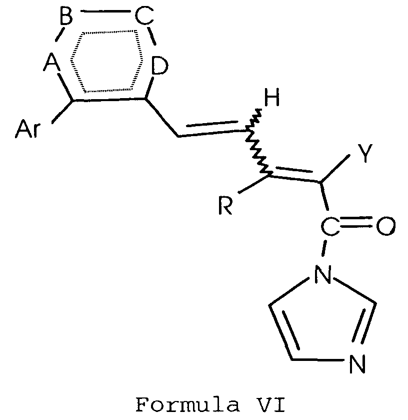 to which is added an amine of the formula R₉NH₂, wherein R₉ is as defined hereinabove, to give an amide of Formula VII: 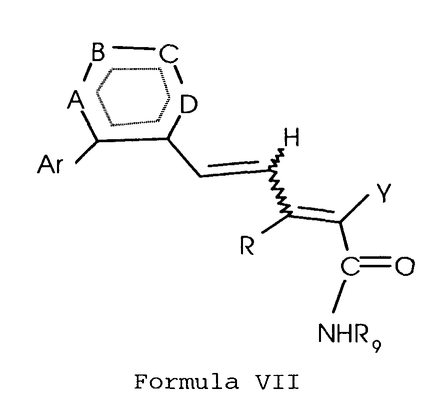 wherein Ar, A, B, C, D, Y, the dotted line (---) and R9 are as defined hereinabove.

According to Scheme 4, a compound of Formula IV: 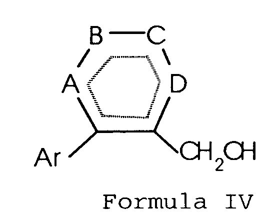 wherein Ar, A, B, C, D and the dotted line (---) are as defined hereinabove; is oxidized with a reagent such as activated MnO₂; in a solvent such as methylene chloride; at 0°C to 40°C for 0.5 to 6.0 hours; to give a compound of Formula VIII: 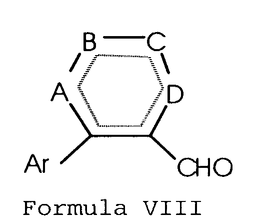 which is reacted with an ylide such as 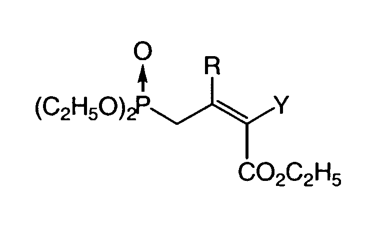 wherein R is hydrogen, methyl, t-butyl or trifluoromethyl; in the presence of a base such as sodium hydride; in an inert solvent such as tetrahydrofuran; to give a compound of Formula I wherein A, B, C, D, Ar, the dotted line (---) and R are as defined hereinabove and X is CO₂C₂H₅.

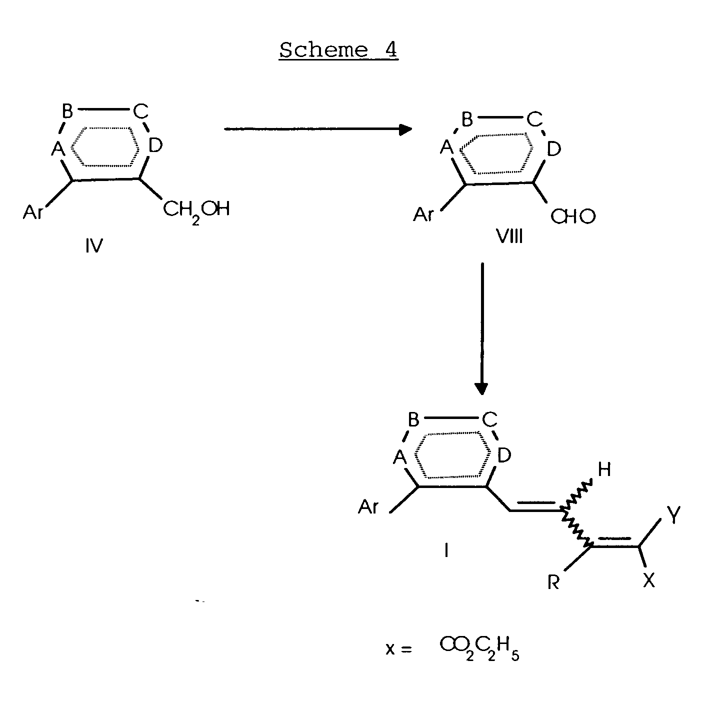

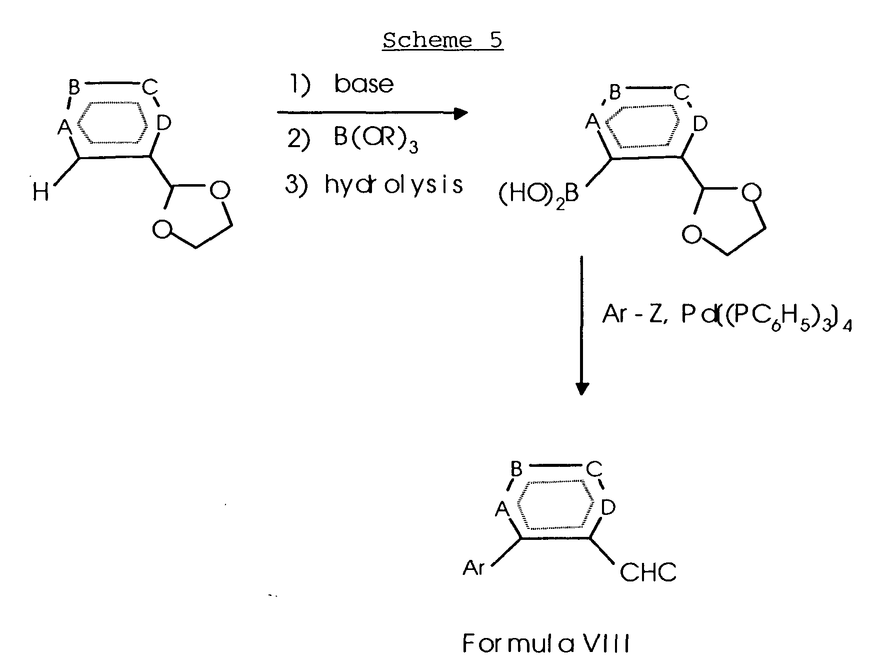

According to Scheme 5, a ketal of the above formula wherein A, B, C, and D are as defined above is reacted with a base such as n-butyllithium in a solvent such as tetrahydrofuran at -78°C to 0°C, followed by the addition of a borate ester such as triisopropylborate and then hydrolysis to a boronic acid. The boronic acid is reacted with an aryl halide of formula ArZ, wherein Z is bromine or iodine in the presence of a palladium(O) catalyst such as tetrakistriphenylphosphine palladium(O) at room temperature, followed by acid hydrolysis to give an aldehyde of formula VIII.

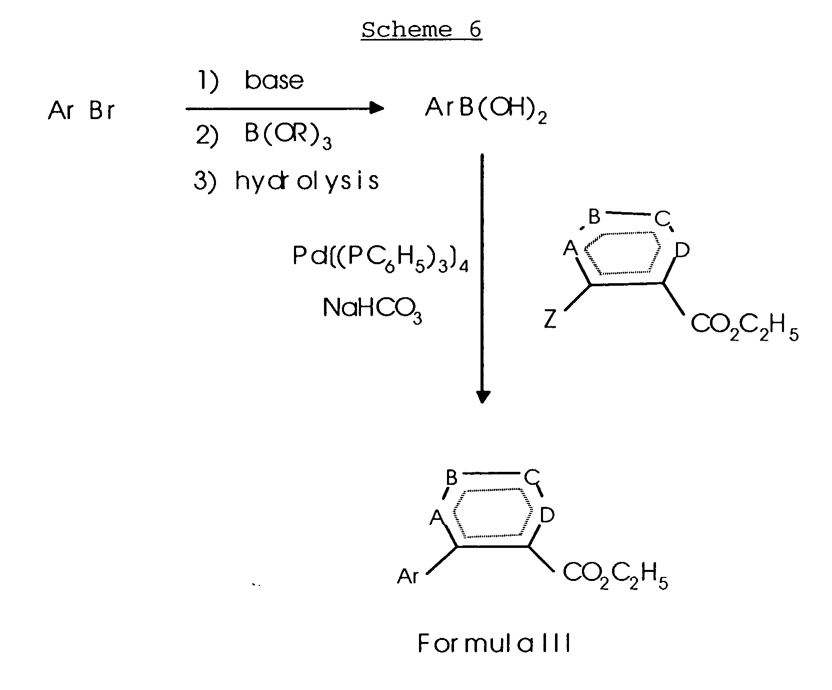

According to Scheme 6, an aryl bromide of formula ArBr, wherein Ar is as defined above is reacted with a base such as n-butyllithium in a solvent such as tetrahydrofuran at -78°C to 0°C, followed by the addition of a borate ester such as triisopropylborate, then hydrolysis to a boronic acid of the formula:

ArB(OH)₂

and this is reacted with a compound of formula: 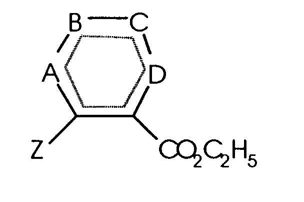 wherein A, B, C, and D are as defined above, and Z is bromine or iodine in a solvent such as dimethoxyethane in the presence of a palladium(O) catalyst, such as tetrakistriphenylphosphine palladium(O) and sodium carbonate to give a compound of Formula III as defined above.

### BIOLOGY

Apolipoprotein (Apo A-I) is the major protein constituent of plasma HDL. Numerous epidemiologic, genetic and biochemical studies have provided strong support for the concept that high plasma HDL concentrations protect against premature atherosclerosis.

The physiological hormones for retinoic acid receptor (RAR) and for retinoic X receptor (RXR) are proposed to be all-*trans*-retinoic acid (RA) and 9-*cis*-retinoic acid (9-*cis* RA), respectively. However, 9*-cis* RA can bind to, and transcriptionally activate the RAR as well. In order for RARs to bind retinoic acid response elements (RAREs) and induce gene transcription effectively, they must form heterodimers with RXRs. However, in the presence of 9-*cis* RA, RXRs can form homodimers that bind and activate specific genes.

The novel compounds of this invention which have the ability to elevate serum levels of apolipo-protein A-I and also HDL in rats, are indicative of therapeutic agents for the treatment of conditions resulting from low HDL, such as atherosclerosis in humans.

Members of the nuclear receptor superfamily, including RXRα, activate transcription by binding to their cognate sites on the DNA located within the vicinity of the start site of the target gene (Evans, R., *Science* **240**, 889 (1988)).

### Biological Results

### Electrophoretic Mobility Shift Assay

The synthetic retinoids are tested in an electrophoretic mobility shift assay (EMSA) in which the ligand dependent binding of RXRα to site A of the Apo A-I gene promoter is monitored (Rottman, J.N., *MCB* **11,** 3814 (1991)). RXRα obtained from E. coli harboring an RXRα-expression plasmid is purified to homogeneity by affinity chromatography. For the EMSA, the purified protein is incubated with a radiolabelled oligonucleo-tide probe containing site A sequences in the absence or presence of the retinoid. RXRα-DNA complexes are resolved from unbound probe by electrophoresis on nondenaturing polyacrylamide gels (Fried, M. and Crothers, D.M., *Nucleic Acid Research* **9**, 6505 (1981)).

9*-cis*-RA, the natural ligand for RXRα, presumably promotes binding of RXRα to the probe, by facilitating homodimer formation (Zhang, X. K., et al, *Nature* **358**, 587 (1992)). The 9-*cis*-RA induced complex is specific as assessed by oligonucleotide competition and antibody supershift (Rottman, J.N., *MCB* **11**, 3814 (1991)). Comparative potencies for test compounds is determined by visual inspection of the intensity of the autoradiograph and are shown in Table 1.

**Table 1**

| **Compound Name** | Potency Relative to **9-*cis*-RA** |
|---|---|
| E, E-3-Methyl-5-[2-(1,1,3,3-tetramethyl-1,3-dihydro-5-isobenzofuranyl)-1-cyclopent-l-yl]-2,4-pentadienoic Acid | 2+ |
| 3-Methyl-TTNEB* (Prior Art Compound) | 1+ |

| | |
|---|---|
| * 3-methyl-TTNEB (Boehm, M., et el, International Publication Number: WO 93/21146, WIPO, October 28, 1993, structure follows) | |

### in Vivo Serum HDL and Apo A-I Assay

Male Wistar rats (190-210g) are used in the study to measure serum levels of HDL. Compounds are suspended in sterile olive oil at a concentration of 20 mg/ml. Rats are bled by retroorbital puncture before starting the study and then given retinoids at a dose of 100mg/kg/day by intraperitoneal injection. Total volume injected is 1 ml with 1 ml olive oil injected into vehicle animals. Rats are injected for 4 days and bled 24 hours after the last injection by heart puncture. Blood is collected in EDTA and the plasma is analyzed for HDL cholesterol, total cholesterol, and Apo A-I.

Agents that induce differentiation have been proposed as alternatives to cytotoscic treatment in cancer therapy. Trans-retinoic acid has been successful in inducing remission in patients with promyelocytic leukemia [R.P. Warrell, et al., New England Journal of Medicine, Vol. 324, 1385-1393, 1991]. Compounds of this invention are tested for their ability to induce differentiation in HL-60 promyelocytic leukemia cells.
CDllb expression: 2.5 x 10⁵HL60 cells are incubated with serial dilutions of drugs for 3 days. Cells are washed with PBA (Dulbecco's PBS (w/out Ca⁺⁺ and Mg⁺⁺), .1% bovine serum albumin and .1% sodium azide) and incubated with 1.6 µg/ml of mouse anti-human CDllb monoclonal antibody (Pharmigen, Cat#30451A) in PBA for 1 hour at 4°C. Cells are washed with PBA and incubated with a 1:50 dilution of goat anti-mouse IgG-FITC (Becton Dickinson, Cat#349031) in PBA for 1 hour at 4°C. Cells are washed twice with PBA, resuspended in PBS and analyzed in the FACSort from Becton Dickinson. 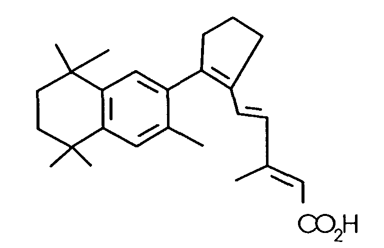 E,E-3-Methyl-5-[2-(5,6,7,8-tetrahydro-3,5,5,8,8-pentamethyl-2-naphthalenyl)-1-cyclopenten-1-yl]-2,4-pentadienoic Acid

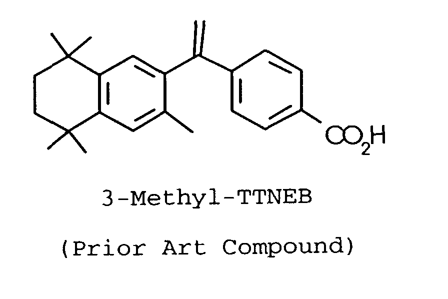

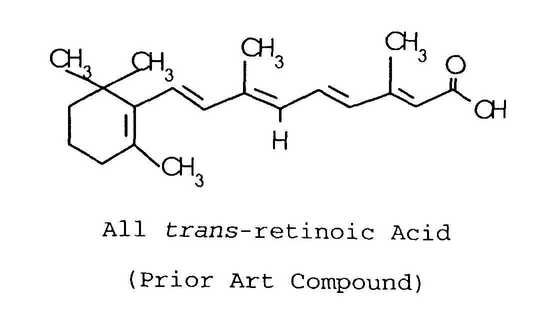

The compounds of Formula I may be obtained as pharmaceutically acceptable salts and esters. The salts are alkali metal salts such as sodium, potassium and lithium; alkaline earth salts such as calcium; ammonium salts; organic salts such as triethyl ammonium, morpholinium, N-methyl-morpholinium and the like. They are made using methods known to those skilled in the art (Richard C. Larock, Comprehensive Organic Transformations, VCH Publishers, 411-415, 1989). It is known to one skilled in the art that an appropriate salt form is chosen based on physical and chemical stability, flowability, hygroscopicity and solubility.

The invention compounds of Formula I may be administered orally to humans in association with a pharmaceutically acceptable carrier, for the treatment and prevention of coronary artery disease, and to protect aginst premature atherosclerosis.

When the compounds of the invention are employed for the above utility, they can be combined with one or more pharmaceutically acceptable carriers, for example, solvents, diluents and the like; and may be administered orally in such forms as tablets, capsules, dispersible powders, granules, or suspensions containing, for example, from about 0.05 to 5% of suspending agent; syrups containing, for example: from about 10 to 50% of sugar; and elixirs containing for example, from about 20 to 50% ethanol and the like, or they may be administered parenterally in the form of sterile injectable solutions or suspensions containing from about 0.05 to 5% suspending agent in an isotonic medium. Such pharmaceutical preparations may contain, for example, from about 25 to 90% of the active ingredient in combination with the carrier, and more usually, between about 5 and 60% by weight.

An effective amount of compound from 2.0 mg/kg of body weight to 100.0 mg/kg of body weight should be administered one to five times per day via any typical route of administration including, but not limited to: oral, parenteral (including subcutaneous, intravenous, intramuscular, intrasternal injection or infusion techniques), topical or rectal, in dosage unit formulations containing conventional non-toxic, pharmaceutically acceptable carriers, adjuvants and vehicles. It will be understood, however, that the specific dose level and frequency of dosage for any particular patient may be varied and will depend upon a variety of factors including: the activity of the specific compound employed, the metabolic stability and length of action of that compound, the age, body weight, general health, sex, diet, mode and time of administra-tion, rate of excretion, drug combination, the severity of the particular condition, and the host undergoing therapy.

These active compounds may be administered orally as well as by intravenous, intramuscular, or subcutaneous routes. Solid carriers include starch, lactose, dicalcium phosphate, microcrystalline cellulose, sucrose and kaolin, which liquid carriers include sterile water, polyethylene glycols, non-ionic surfactants and edible oils such as corn, peanut and sesame oils, as are appropriate to the nature of the active ingredient and the particular form of admini-stration desired. Adjuvants customarily employed in the preparation of pharmaceutical compositions may be advantageously included, such as flavoring agents, coloring agents, preserving agents, and antioxidants, for example: vitamin E, ascorbic acid, BHT, and BHA.

The preferred pharmaceutical compositions from the standpoint of ease of preparation and administration are solid compositions, particularly tablets and hard-filled or liquid-filled capsules. Oral administration of the compounds is preferred.

These active compounds may also be administered parenterally or intraperitoneally. Solutions or suspensions of these active compounds as a free base or pharmacologically acceptable salt can be prepared in glycerol, liquid, polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions, and sterile powders for the extemporaneous preparation of sterile injectible solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example: water, ethanol, polyol (e.g., glycerol, propylene glycol and liquid polyethylene glycol), suitable mixtures thereof, and vegetable oil.

The compounds may also be encapsulated in liposomes to allow an intravenous administration of the drug. The liposomes suitable for use in this invention are lipid vesicles and may include plurilamellar lipid vesicles, small sonicated multilamellar vesicles, reverse phase evaporation vesicles, large multilamellar vesicles and the like wherein the lipid vesicles are formed of one or more phospholipids such as phospha-tidylcholine, phosphatidylglycerol, sphingomyelin, phospholactic acid and the like.

The following examples describe in detail the chemical synthesis of representative compounds of the present invention. The procedures are illustrations, and the invention should not be construed as being limited by chemical reactions and conditions that they express. No attempt has been made to optimize the yields obtained in these reactions, and it would be obvious to one skilled in the art that variations in reaction times, temperatures, solvents, and/or reagents could increase the yields.

Preparative Examples A and B illustrate a process for preparing compounds of formula (I).

### Preparative Example A

### Z,E and E,E-3-Methyl-5-[2-(5,6,7,8-tetrahydro-5,5,8,8-tetramethyl-2-naphthalenyl)-1-cyclohexen-1-yl]-2,4-pentadienoic Acid Ethyl Ester

To a stirred solution of 2.0 g of 2-bromo-(5,6,7,8-tetrahydro-5,5,8,8-tetramethyl)naphthalene in 20 ml of anhydrous tetrahydrofuran at -78°C is added 9 ml of 1.7M tert-butyllithium in pentane, followed by the addition of 15 ml of zinc chloride, 0.5M in tetrahydrofuran. The mixture is allowed to warm to room temperature, 0.39 g of tetrakistriphenyl phosphine-palladium(0) and 1.0 g of ethyl 2-trifluoromethane-sulfonyloxycyclohexen-1-ylcarboxylate in 5 ml of tetrahydrofuran is added and the resulting solution is stirred for 2 hours at the reflux temperature of the solvent. The reaction is cooled to room temperature, 50 ml of diethyl ether is added and the layers are separated. The organic layer is washed with water, aqueous sodium bicarbonate, saturated sodium chloride, and dried over sodium sulfate. Evaporation of the solution, followed by chromatography (silica gel: hexane/diethyl ether 4:1) gives 1.5 g of 2-(5,6,7,8-tetrahydro-5,5,8,8-tetramethyl-2-naphthalenyl)-1-cyclohexene-1-carboxylic acid, ethyl ester as a colorless solid.

A solution of 1.48 g of the above isolated solid in 15 ml of diethyl ether is added dropwise at 0°C to 10 ml of 1.ON lithium aluminum hydride in anhydrous tetrahydrofuran, followed by stirring at room temperature for 15 minutes. Water is added dropwise to the cooled reaction mixture. The reaction is extracted with diethyl ether. The organic layers are combined, dried over sodium sulfate and evaporated to give the alcohol as an oil. The oil is dissolved in 60 ml of methyl alcohol to which is added 1.5 g of triphenyl- phosphine hydrobromide and the reaction mixture is stirred at room temperature for 17 hours. The solvent is removed *in vacuo,* and the residue is washed with diethyl ether to give the corresponding phosphonium bromide, and then this is dissolved in 25 ml of dry methylene chloride, cooled under argon to 0°C, and sodium ethoxide and 0.7 ml of ethyl 3-methyl-4-oxocrotonate are added. The mixture is stirred at 0°C for 1 hour and quenched with water. The methylene chloride extract is dried over sodium sulfate, evaporated to a red oil and the oil is purified by chromatography (silica gel: hexane/diethyl ether 9:1) to give 1.1 g of a 15:2 mixture of E,E and Z,E esters.

### Preparative Example B

### E,E-3-Methyl-5-[2-(5,6,7,8-tetrahydro-5,5,8,8-tetramethyl-2-naphthalenyl)-1-cyclohexen-1-yl]-2,4-pentadienoic Acid

The mixed (15:2) ester product from Example 1 is combined with 5 ml of 2N aqueous potassium hydroxide in 10 ml of methyl alcohol and stirred at reflux temperature for 3 hours. The reaction mixture is cooled to room temperature, poured into a mixture of ice and methylene chloride, and acidified to pH 3 with 3N hydrochloric acid. The organic layers are combined, dried over sodium sulfate and evaporated to give a light yellow solid. The solid is recrystallized form absolute ethyl alcohol to give 0.5 g of the desired compound as colorless crystals.
mp 198-199 °C;
¹H NMR(CDCl₃):δ 1.24(s,6H), 1.26(s,6H), 1.69(s,4H), 1.76-1.82(m, 4H), 2.10(s, 3H), 2.32-2.39(m, 2H), 2.46-2.50(m, 2H), 5.77(s, 1H), 6.25(d, J=16.0Hz, 1H), 6.86(d, J=16.0Hz, 1H), 6.95(d, J=7.0Hz, 1H), 7.06(s, 1H), 7.28 (d,J=7.0Hz, 1H) 13_{C} NMR(CDCl₃) ppm downfield from TMS: 13.90, 22.52, 22.78, 22.91, 25.66, 31.80, 31.90, 33.05, 34.10, 34.22, 35.06, 35.14, 116.93, 125.46, 126.16, 127.56, 128.58, 129.96, 136.46, 139.26, 143.66, 144.15, 144.49, 156.33, 172.39.
IR(KBr): 3054, 2959, 2928, 2861, 1593, 1679, 1595, 1491, 1457, 1363, 1349, 1262, 1188, 963, 878 cm⁻¹.
MS(CI) :m/z 379.
UV(in CH₃OH): 317 nM.

### Example 1

### 5-Bromo-1,1,3,3-tetramethyl-1,3-dihydroisobenzofuran

A solution of 5.0 g of 4-bromophthalic acid dissolved in 200 ml of absolute methyl alcohol is saturated with dry hydrogen chloride gas and allowed to stir at room temperature for 20 hours. The reaction mixture is evaporated *in vacuo,* kept under high vacuum overnight to give 5.13 g of dimethyl-4-bromophthalate as a pale yellow oil.
¹HNMR(CDCl₃):δ 3.90(s,3H); 3.92(s,3H); 7.62(d,J=8.3Hz,1H);
7.67(dd,J=8.3Hz,1.8Hz,1H); 7.84(d,J=1.8Hz,1H).

A mixture of 27.3 g of the above product dissolved in 100 ml of tetrahydrofuran is cooled in an ice bath. To this cooled mixture is added, dropwise over 30 minutes, 200 ml of 3.0M methylmagnesium chloride in tetrahydrofuran. After the addition is completed, the reaction is heated at reflux temperature for 24 hours, quenched into 400 ml of saturated ammonium chloride, and extracted with diethyl ether. The combined organic layers are washed with saturated sodium chloride, dried over sodium sulfate, filtered and evaporated to give the crude product as an oil. The crude product is crystallized with hexane to give 9.55 g of 2,2'-(4-bromo-1,2-phenylene)bis(2-propanol).
¹HNMR (CDCl₃):δ 1.69(s,6H); 1.70(s,6H), 4.97(brs,2H);
7.18(d,J=8.6Hz,lH); 7.28(dd,J=8.7Hz,2.22Hz,1H) ;
7.44(d,J=2.Hz,1H).

To 26 ml of 60% sulfuric acid is added 2.92 g of the above diol product. The mixture is heated at 50°C for 1 hour. The reaction mixture is poured into water and extracted with hexane. The combined hexane layers are washed with saturated sodium bicarbonate, saturated sodium chloride, dried over sodium sulfate, filtered through a short pad of hydrous magnesium silicate and evaporated to give 2.47 g of the desired title compound as a white solid.
¹HNMR (CDCl₃) :δ 1.49(s,6H); 1.50(s,6H); 6.96(d,J=8.0Hz,1H) 7.22(d,J=1.6Hz,1H); 7.39(dd,1.7Hz,1H).

### Example 2

### E,E,and Z,E-3-Methyl-5-[2-(1,1,3,3-tetramethyl-1,3-dihydro-5-isobenzofuranyl)-1-cyclohexen-1-yl]-2,4-pentadienoic Acid, Ethyl Ester

The title compound, as a mixture of E,E and Z,E isomers, is prepared by the procedure of Preparative Example A using the product from Example 1.

### Example 3

### E,E-3-Methyl-5-[2-(1,1,3,3-tetramethyl-1,3-dihydro-5isobenzofuranyl)-1-cyclohexen-1-yl]-2,4-pentadienoic Acid

The title compound is prepared by the procedure of Preparative Example B using the product from Example 2.

### Example 4

### E,E-3-Methyl-5-[2-(1,1,3,3-tetramethyl-1,3-dihydro-5-isobenzofuranyl)-1-cyclopent-1-yl] -2,4-pentadienoic Acid

Following the procedure of Preparative Examples A and B, the title compound is prepared using the product from Example 1 and ethyl trifluoromethanesulfonyloxycyclopent-1-yl carboxylate to give the desired product as colorless crystals.
mp = 211 - 212°C.
¹HNMR (CDCl₃):δ 1.47(s,6H), 1.54(s,6H), 1.98-2.03(m,2H), 2.27(s,3H), 2.71-2.76(m,2H), 2.85-2.92(m,2H), 5.85(s,1H), 6.30(d, J=16.0Hz,1H), 7.02(s,1H), 7.08(d, J=16.0Hz,1H), 7.11 (d, J=16.0Hz,1H), 7.23(d, J=16.0Hz,1H).
MS(CI): m/z 353 (M⁺+H).

### Example 6

### E,E-3-Methyl-5-[2-(2-(3,5,5,8,8-pentamethyl-5,6,7,8-tetrahydro-2-naphthalenyl)-1-cyclopenten-1-yl]-2,4-pentadienol

a) Z,E- and E,E-3-Methyl-5-[2-(2-(3,5,5,8,8-pentamethyl-5,6,7,8-tetrahydro-2-naphthalenyl)-1-cyclopenten-1-y1]-2,4-pentadienoic Acid Ethyl Ester
   The title compound is prepared by the procedure of Preventative Example A using ethyl 2-trifluoromethanesulfonyloxycyclopenten-1-yl carboxylate to give a 17:5 mixture of isomers which can be separated by chromatography into the individual isomers.
b) The product from step a) is reduced with lithium aluminum hydride in diethyl ether. The reaction mixture is quenched with water and the resultant mixture is extracted with ether, and on evaporation the desired compound is obtained.

### Examples 7 - 8

The product of the indicated Example is reduced as in Example 6 to the corresponding alcohol as outlined in Table:

| Example No | Product of Example | Name of Product |
|---|---|---|
| 7 | 4 | E,E-3-Methyl-5-[2-(1,3-dihydro-1,1,3,3-tetramethyl-5-isobenzofuranyl)-1-cyclopent-1-yl]-2,4-pentadienol |
| 8 | 5 | E,E-3-Methyl-5-[2-(1,3-dihydro-1,1,3,3,6-pentamethyl-5-isobenzofuranyl)-1-cyclopent-1-yl]-2,4-pentadienol |

### Example 9

### E,E-3-Methyl-5-[2-(1,1,3,3,6-pentamethyl-1,3-dihydro-5-isobenzofuranyl)-1-cyclopent-1-yl]-2,4-pentadienoic Acid

Using the procedure of Example 4 and substituting 5-bromo-1,1,3,3, 6-pentamethylisobenzofuran, there is obtained the title compound.

### Example 10

### E,E-3-Methy1-5-[2-(1,1,3,3,6-pentamethyl-1,3-dihydro-5-isobenzofuranyl)-1-cyclohexen-1-yl]-2,4-pentadienoic Acid

Using the procedure of Example 3 and substituting 5-bromo-1,1,3,3,6-pentamethylisobenzofuran, there is obtained the title compound.
¹HNMR (CDCl₃) δ 1.47(s,3H), 1.50(s,3H), 1.52(s,6H), 1.70-1.86(m,4H), 1.98(s,3H), 2.16(s,3H), 2.29-2.39(m,4H), 5.75(s,1H), 6.21(d,J=16.0Hz,1H), 6.41(d,J=16.0Hz,1H), 6.72(s,1H), 6.91(s,1H).

## Claims

1. A compound of Formula I: 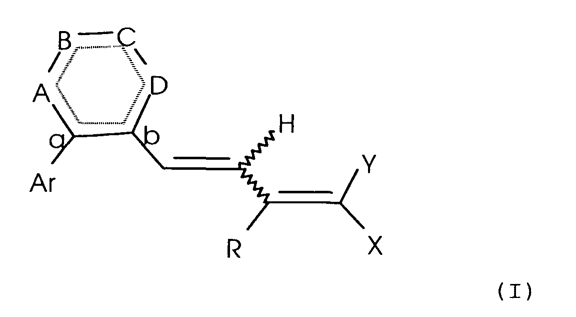 wherein:
A, B and C are CH, CH₂, O, or S wherein no more than one heteroatom is present;
D is (CH)ₘ, and m is an integer 0 to 1; or D is (CH₂)ₙ, and
n is an integer 0 to 2;
the dotted line, -----, represents the presence or absence of a double bond whereby if only one double bond is present, it is disposed to the a-b position; or if multiple double bonds are present they are in a conjugated position to produce an aromatic ring;
R is hydrogen, methyl, ethyl, t-butyl, or trifluoromethyl;
Ar is:
a moiety of the formula: 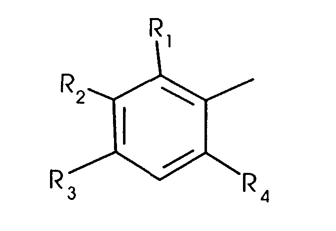
wherein R₁, R₂, R₃, and R₄ are hydrogen, (C₁-C₃)alkyl, (C₁-C₃) alkoxy or trifluoromethyl;
or a moiety of the formula: 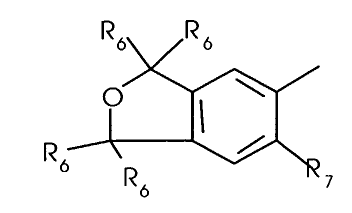
wherein R₆ is hydrogen, methyl or ethyl;
R₇ is hydrogen, methyl or ethyl; Y is hydrogen, and
X is CH₂OH, CHO, CO₂H, CN, CH₂CONH₂, or a moiety of the formula: 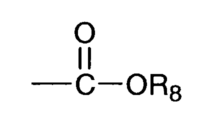
and R₈ is straight or branched (C₁-C₈) alkyl, or 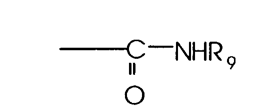
wherein R₉ is hydrogen, straight or branched (C₁-C₁₀)alkyl, glycosyl, 2-methoxyethyl, 2-dimethyl-aminoethyl, (2,3 or 4)-pyridyl, or (2,3 or 4)-pyridylmethyl; or X and Y taken together form the thiazolidinedione ring of the formula: 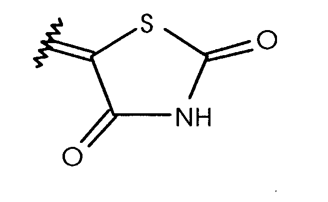 and the pharmaceutically acceptable salts and esters.

2. A compound of Formula I as defined in claim 1 wherein:
A, B, C, D, the dotted line, R and X are as defined in claim 1;
Y is hydrogen and
Ar is a moiety of the formula: 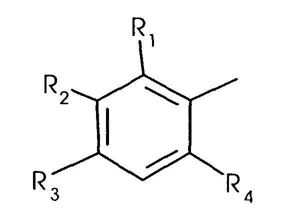
wherein R₁, R₂, R₃, and R₄ are hydrogen, (C₁-C₃) alkyl, (C₁-C₃)alkoxy or trifluoromethyl;
and the pharmaceutically acceptable salts and esters.

3. A compound of Formula I as defined in claim 1 wherein:
A is CH or CH₂;
B, C, D, R, X and the dotted line are as defined in claim 1;
Ar is a moiety of the formula: 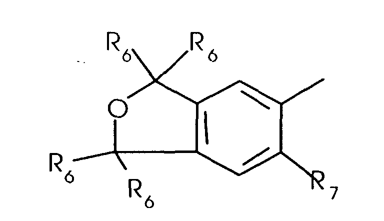
wherein R₆ and R₇ are as defined in claim 1; and
Y is hydrogen,
and the pharmaceutically acceptable salts and esters.

4. A compound according to Claim 1 which is E,E-3-Methyl-5-[2-(1,1,3,3-tetramethyl-1,3-dihydro-5-isobenzofuranyl)-1-cyclopent-1-yl]-2,4-pentadienoic Acid.

5. Use of a compound according to any one of Claims 1 to 4 in the preparation of a medicament for the treatment or prevention of coronary artery disease in a mammal.

6. Use of a compound of according to any one of Claims 1 to 4 in the preparation of a medicament for increasing plasma HDL levels in a mammal.

7. Use of a compound according to any one of Claims 1 to 4 in the preparation of a medicament for prevention of atherosclerosis in a mammal.

8. Use of a compound according to any one of Claims 1 to 4 in the preparation of a medicament for treating cancers by induction of tumor cell differentiation in a mammal.

9. A compound of formula I as defined in any one of Claims 1 to 4 for use as a pharmaceutical.

10. A process for preparing a compound of formula I as defined in claim 1 or a pharmaceutically acceptable salt or ester thereof which comprises one of the following:
a) reacting a compound of formula 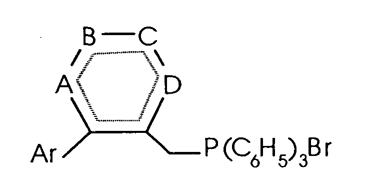 with an aldehyde of formula 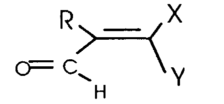 in which formulae Ar, A, B, C, D, R, and the dotted line are as defined in claim 1, X is CO₂R₈ and Y is hydrogen to give a corresponding compound of formula I; or
b) hydrolysing a compound of formula I where X is COR₈ to give a carboxylic acid wherein X is CO₂H;
or
c) reducing a compound of formula I where X is CO₂R₈ or CO₂H to give a compound of formula I wherein X is CH₂OH;
or
d) reacting a compound of formula I wherein X is CO₂H with an activating reagent and followed by an amine of formula R₉NH₂ to give an amide of formula I wherein X is CONHR₉;
or
e) reacting a compound of formula VIII 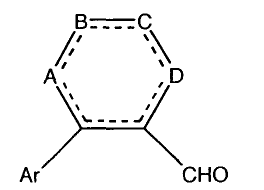 with a ylide of formula 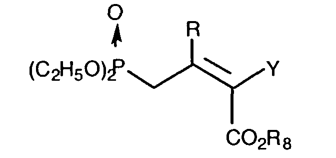 to give a corresponding compound of formula I wherein Ar, A, B, C, D, R, Y and R₈ are as defined above;
or
f) oxidising a compound of formula I wherein X is CH₂OH to give a compound of formula I wherein X is CHO;
or
g) dehydrating a compound of formula I wherein X is CONH₂ to give a compound of formula I wherein X is CN, eg using P₂O₅; or
h) reacting a compound of formula I wherein X is CN with sodium azide to give a compound of formula I wherein X is 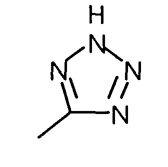 or
i) reacting an aldehyde of formula 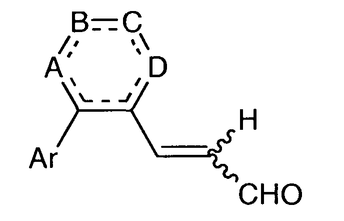 wherein Ar, A, B, C, D and the dotted line are as defined above with 2,4-thiazolidinedione to give a compound of formula I wherein X and Y taken together represent

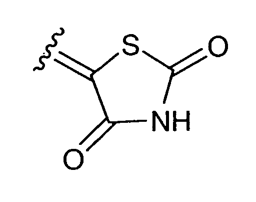

11. A process for preparing a compound of formula I according to Claim 1, which comprises reacting a compound of the formula ArBr or ArI with an alkyllithium, in an inert solvent, at -78°C to +30°C, followed by adding ZnCl₂; to give a compound of formula:
ArZnCl
reacting the ArZnCl with a compound of Formula II: 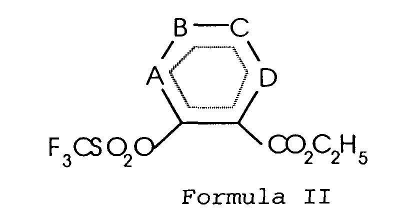 in the presence of a palladium(0) catalyst, in an inert solvent, at 10°C to 60°C; to obtain a compound of Formula III: 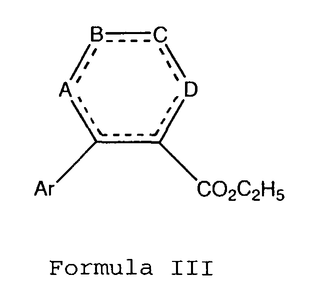 reducing the compound of Formula III with a hydride reducing agent in an inert solvent at 0°C to 60°C; to give a compound of Formula IV: 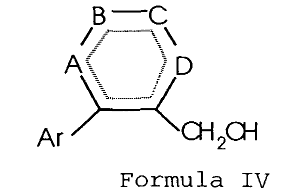 reacting the compound of Formula IV with a trisubstituted phosphine hydrobromide to give a compound of Formula V: 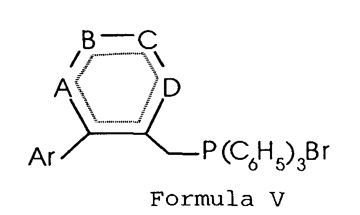 reacting the compound of Formula V with a base, in an inert solvent,at 0°C; followed by addition of an aldehyde of the formula: 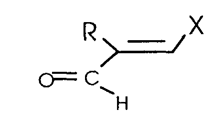 wherein R is as defined in Claim 1; and
X is CO₂R₈ and R₈ is as defined in Claim 1; to obtain a compound of Formula I.

12. A process for preparing a compound of formula I defined in Claim 1 wherein X is -CHO; which comprises reacting a compound of Formula I wherein X is -CO₂H or-CO₂R₈ and R₈ is as defined in Claim 1; with a hydride reducing agent, in an inert solvent, at 0°C to 60°C; to obtain a compound of Formula I wherein X is -CH₂OH; treating the compound of Formula I wherein X is CH₂OH with an oxidizing agent to give a compound of Formula I wherein X is -CHO.

13. A process for preparing a compound according to Claim 1 wherein X is -C(O)-NHR₉ wherein R₉ is as defined in Claim 1;
which comprises reacting a compound of Formula I wherein X is -CO₂R₈ and R₈ is as defined in Claim 1: with a base under hydrolysis conditions at 30°C to 100°C, followed by acidification with a mineral acid; to obtain a compound of Formula I wherein X is -CO₂H; reacting the compound of Formula I wherein X is -CO₂H with an activating reagent in an inert solvent, at 0°C to 25°C; to give an intermediate; adding an amine of the formula R₉NH₂ to the intermediate, wherein R₉ is as defined in Claim 1; to obtain a compound of Formula I.

14. The process for preparing a compound according to Claim 1 wherein X is -CO₂R₈ and R₈ is as defined in Claim 1; which comprises reacting a compound of Formula IV: 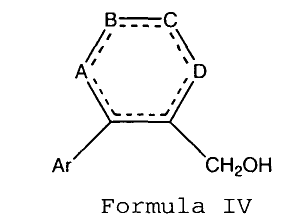 wherein Ar and Y are as defined hereinabove; with an oxidizing agent, in an inert solvent, at 0°C to 40°C; to give a compound of Formula VIII: 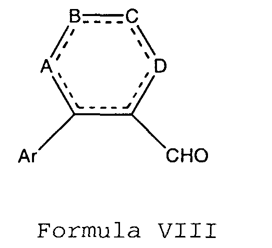 reacting the compound of Formula VIII with an ylide of the formula: 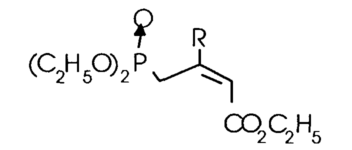 in the presence of a base in an inert solvent; to give a compound of Formula I.

15. A pharmaceutical composition which comprises a compound as claimed in any one of Claims 1 to 5 and a pharmaceutically acceptable carrier.

## Patentansprüche

1. Verbindung der Formel I: 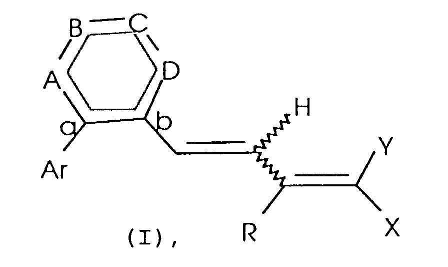 worin:
A, B und C für CH, CH₂, O oder S stehen, worin nicht mehr als ein Heteroatom vorhanden ist;
D für (CH)ₘ steht und m eine ganze Zahl 0 bis 1 ist; oder D für (CH₂)ₙ steht und n eine ganze Zahl 0 bis 2 ist;
die gestrichelte Linie, -----, die Anwesenheit oder Abwesenheit einer Doppelbindung darstellt, wobei falls nur eine Doppelbindung vorhanden ist, diese der a-b Position zugeordnet ist; oder falls mehrere Doppelbindungen vorhanden sind, diese sich in einer konjugierten Position befinden, um einen aromatischen Ring zu bilden;
R für Wasserstoff, Methyl, Ethyl, t-Butyl oder Trifluormethyl steht;
Ar für:
eine Komponente der Formel: 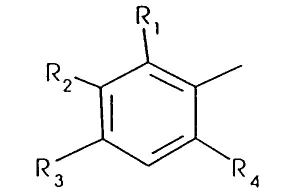
worin R₁, R₂, R₃ und R₄ für Wasserstoff, (C₁-C₃)Alkyl, (C₁-C₃)Alkoxy oder Trifluormethyl stehen,
oder eine Komponente der Formel: 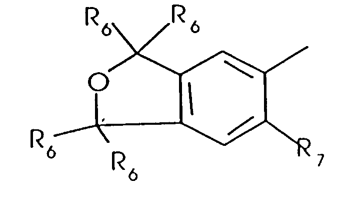 steht, worin R6 Wasserstoff, Methyl oder Ethyl darstellt; R₇ Wasserstoff, Methyl oder Ethyl darstellt; Y Wasserstoff darstellt und X für CH₂OH, CHO, CO₂H, CN, CH₂CONH₂, oder eine Komponente der Formel: 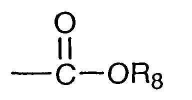 steht und R₈ gerades oder verzweigtes (C₁-C₈)Alkyl darstellt, oder 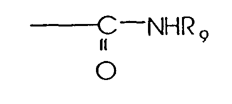
worin R₉ für Wasserstoff, gerades oder verzweigtes (C₁-C₁₀)Alkyl, Glycosyl, 2-Methoxyethyl, 2-Dimethyl-aminoethyl, (2,3 oder 4)-Pyridyl oder (2,3 oder 4)-Pyridylmethyl steht; oder X und Y zusammengenommen den Thiazolidindionring der Formel: 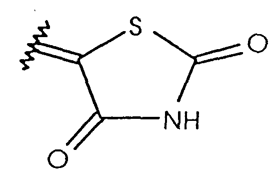 bilden, und die pharmazeutisch annehmbaren Salze und Ester.

2. Verbindung der Formel I, wie in Anspruch 1 definiert, worin:
A, B, C, die gestrichelte Linie, R und X wie in Anspruch 1 definiert sind;
Y Wasserstoff darstellt und
Ar für eine Komponente der Formel: 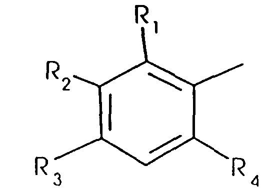 steht, worin R₁, R₂, R₃ und R₄ für Wasserstoff, (C₁-C₃)Alkyl, (C₁-C₃) Alkoxy oder Trifluormethyl stehen;
und die pharmazeutisch annehmbaren Salze und Ester.

3. Verbindung der Formel 1, wie in Anspruch 1 definiert, worin:
A für CH oder CH₂ steht;
B, C, D, R, X und die gestrichelte Linie wie in Anspruch 1 definiert sind;
Ar für eine Komponente der Formel: 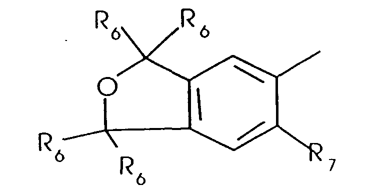 steht, worin R₆ und R₇ wie in Anspruch 1 definiert sind, und Y Wasserstoff darstellt,
und die pharmazeutisch annehmbaren Salze und Ester.

4. Verbindung gemäß Anspruch 1, welche E,E-3-Methyl-5-[2-(1,1,3,3-tetramethyl-1,3-dihydro-5-isobenzofuranyl)-1-cyclopent-1-yl]-2,4-pentadiensäure ist.

5. Verwendung einer Verbindung gemäß einem der Ansprüche 1 bis 4 bei der Herstellung einer Arznei für die Behandlung oder Prävention koronarer Arterienerkrankung in einem Säuger.

6. Verwendung einer Verbindung gemäß einem der Ansprüche 1 bis 4 bei der Herstellung einer Arznei zum Erhöhen von Plasma-HDL-Levels in einem Säuger.

7. Verwendung einer Verbindung gemäß einem der Ansprüche 1 bis 4 bei der Herstellung einer Arznei zur Prävention von Atherosklerose in einem Säuger.

8. Verwendung einer Verbindung gemäß einem der Ansprüche 1 bis 4 bei der Herstellung einer Arznei zum Behandeln von Krebsen durch Induktion von Tumorzellen-Differentiation in einem Säuger.

9. Verbindung der Formel I, wie in einem der Ansprüche 1 bis 4 definiert, zur Verwendung als Pharmazeutikum.

10. Verfahren zum Herstellen einer Verbindung der Formel I, wie in Anspruch 1 definiert, oder eines pharmazeutisch annehmbaren Salzes oder Esters davon, welches eines der folgenden umfasst:
a) Umsetzen einer Verbindung der Formel 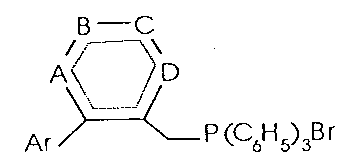 mit einem Aldehyd der Formel 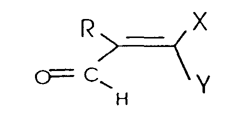 in welchen Formeln Ar, A, B, C, D, R und die gestrichelte Linie wie in Anspruch 1 definiert sind, X für CO₂R₈ steht und Y Wasserstoff darstellt, um eine entsprechende Verbindung der Formel I zu ergeben; oder
b) Hydrolysieren einer Verbindung der Formel I, worin X für COR₈ steht, um eine Carbonsäure zu ergeben, worin X für CO₂H steht; oder
c) Reduzieren einer Verbindung der Formel I, worin X für CO₂R₈ oder CO₂H steht, um eine Verbindung der Formel I zu ergeben, worin X für CH₂OH steht; oder
d) Umsetzen einer Verbindung der Formel I, worin X für CO₂H steht, mit einem aktivierenden Reagenz und gefolgt von einem Amin der Formel R₉NH₂, um ein Amid der Formel I zu ergeben, worin X für CONHR₉ steht; oder
e) Umsetzen einer Verbindung der Formel VIII 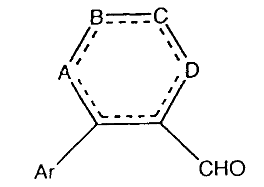 mit einem Ylid der Formel 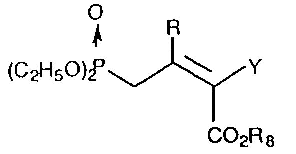 um eine entsprechende Verbindung der Formel I zu ergeben, worin Ar, A, B, C, D, R, Y und R₈ wie oben definiert sind; oder
f) Oxidieren einer Verbindung der Formel I, worin X für CH₂OH steht, um eine Verbindung der Formel I zu ergeben, worin X für CHO steht; oder
g) Dehydrieren einer Verbindung der Formel I, worin X für CONH₂ steht, um eine Verbindung der Formel I zu ergeben, worin X für CN steht, z.B. unter Verwendung von P₂O₅; oder
h) Umsetzen einer Verbindung der Formel I, worin X für CN steht, mit Natriumazid, um eine Verbindung der Formel I zu ergeben, worin X für 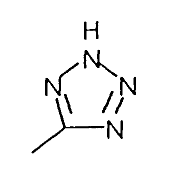 steht; oder
i) Umsetzen eines Aldehyds der Formel 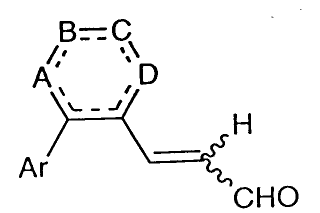 worin Ar, A, B, C, D und die gestrichelte Linie wie oben definiert sind, mit 2,4-Thiazolidindion, um eine Verbindung der Formel I zu ergeben, worin X und Y zusammengenommen 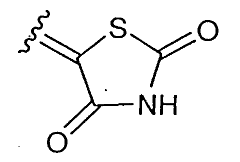 darstellen.

11. Verfahren zum Herstellen einer Verbindung der Formel I gemäß Anspruch 1, welches umfasst: Umsetzen einer Verbindung der Formel ArBr oder ArI mit einem Alkyllithium in einem inerten Lösungsmittel bei -78°C bis +30°C, gefolgt von Zugabe von ZnCl₂, um eine Verbindung der Formel
ArZnCl
zu ergeben, Umsetzen des ArZnCl mit einer Verbindung der Formel II: 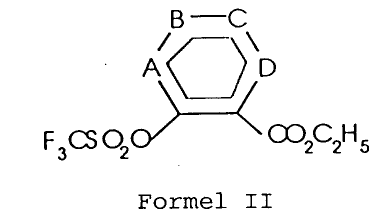 in Anwesenheit eines Palladium(0)-Katalysators in einem inerten Lösungsmittel bei 10°C bis 60°C; um eine Verbindung der Formel III: 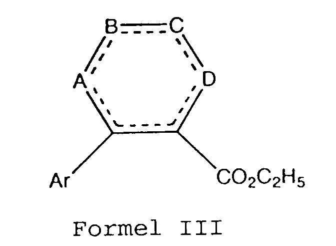 zu erhalten, Reduzieren der Verbindung der Formel III mit einem Hydrid-Reduktionsmittel in einem inerten Lösungsmittel bei 0°C bis 60°C, um eine Verbindung der Formel IV: 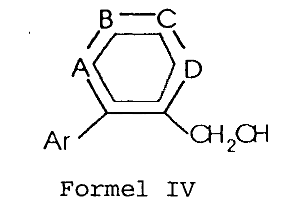 zu ergeben, Umsetzen der Verbindung der Formel IV mit einem trisubstituierten Phosphinhydrobromid, um eine Verbindung der Formel V: 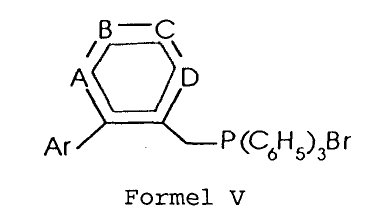 zu ergeben, Umsetzen der Verbindung der Formel V mit einer Base in einem inerten Lösungsmittel bei 0°C; gefolgt von Zugabe eines Aldehyds der Formel: 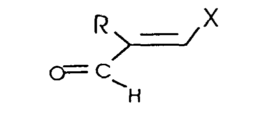 worin R wie in Anspruch 1 definiert ist; und X für CO₂R₈ steht und R₈ wie in Anspruch 1 definiert ist; um eine Verbindung der Formel I zu erhalten.

12. Verfahren zum Herstellen einer Verbindung der Formel I, definiert in Anspruch 1, worin X für -CHO steht; welches umfasst: Umsetzen einer Verbindung der Formel I, worin X für -CO₂H oder -CO₂R₈ steht und R₈ wie in Anspruch 1 definiert ist; mit einem Hydrid-Reduktionsmittel in einem inerten Lösungsmittel bei 0°C bis 60°C; um eine Verbindung der Formel I zu erhalten, worin X für -CH₂OH steht; Behandeln der Verbindung der Formel I, worin X für CH₂OH steht, mit einem Oxidationsmittel, um eine Verbindung der Formel I zu ergeben, worin X für -CHO steht.

13. Verfahren zum Herstellen einer Verbindung gemäß Anspruch 1, worin X für -C(O)-NHR₉ steht, worin R₉ wie in Anspruch 1 definiert ist; welches umfasst: Umsetzen einer Verbindung der Formel I, worin X für -CO₂R₈ steht und R₈ wie in Anspruch 1 definiert ist; mit einer Base unter Hydrolysebedingungen bei 30°C bis 100°C, gefolgt von Säuerung mit einer Mineralsäure; um eine Verbindung der Formel I zu erhalten, worin X für -CO₂H steht; Umsetzen der Verbindung der Formel I, worin X für -CO₂H steht, mit einem aktivierenden Reagenz in einem inerten Lösungsmittel bei 0°C bis 25°C; um einen Zwischenstoff zu ergeben; Zugabe von einem Amin der Formel R₉NH₂ zu dem Zwischenstoff, worin R₉ wie in Anspruch 1 definiert ist; um eine Verbindung der Formel I zu erhalten.

14. Verfahren zum Herstellen einer Verbindung gemäß Anspruch 1, worin X für -CO₂R₈ steht und R₈ wie in Anspruch 1 definiert ist; welches umfasst: Umsetzen einer Verbindung der Formel IV: 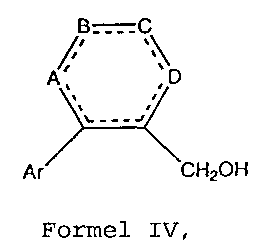 worin Ar und Y wie vorstehend definiert sind; mit einem Oxidationsmittel in einem inerten Lösungsmittel bei 0°C bis 40°C; um eine Verbindung der Formel VIII: 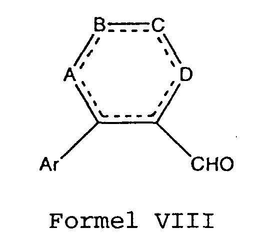 zu ergeben, Umsetzen der Verbindung der Formel VIII mit einem Ylid der Formel: 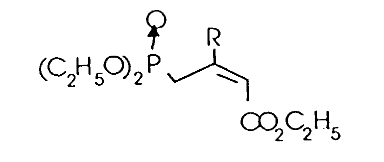 in Anwesenheit einer Base in einem inerten Lösungsmittel; um eine Verbindung der Formel I zu ergeben.

15. Pharmazeutische Zusammensetzung, welche umfasst: eine Verbindung wie in einem der Ansprüche 1 bis 5 beansprucht und einen pharmazeutisch annehmbaren Träger.

## Revendications

1. Composé de formule I 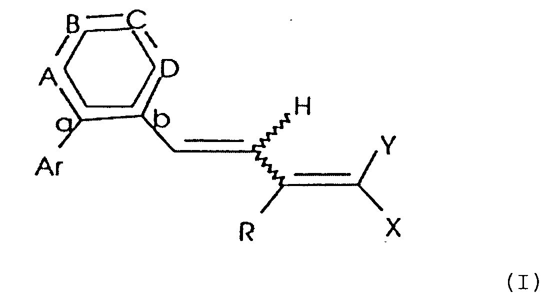 dans laquelle
A, B et C sont CH, CH₂, O ou S dans lequel pas plus d'un hétéroatome n'est présent ;
D est (CH)ₘ et m est un entier de 0 à 1 ; ou D est (CH₂)ₙ et n est un entier de 0 à 2 ;
la ligne en pointillés, ---, représente la présence ou l'absence d'une double liaison par quoi si seulement une double liaison est présente, elle est disposée à la position a-b ; ou si des liaisons doubles multiples sont présentes, elles sont dans une position conjuguée pour produire un cycle aromatique ;
R est hydrogène, méthyle, éthyle, t-butyle ou trifluorométhyle ;
Ar est : une partie de la formule : 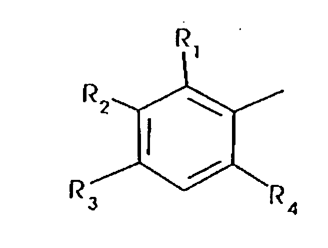 dans laquelle R₁, R₂, R₃ et R₄ sont hydrogène, alkyle en (C₁-C₃), alkoxy en (C₁-C₃) ou trifluorométhyle ;
ou une partie de la formule : 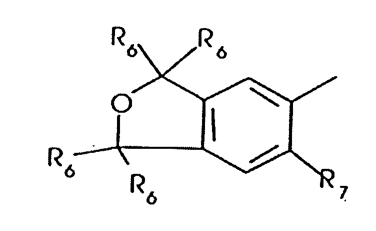
dans laquelle R₆ est hydrogène, méthyle ou éthyle ;
et R₇ est hydrogène, méthyle ou éthyle ; Y est hydrogène, et X est CH₂OH, CHO, CO₂H, CN, CH₂CONH₂ ou une partie de la formule 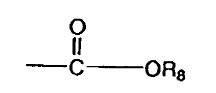
et R₈ est un alkyle linéaire ou ramifié en (C₁-C₈), ou 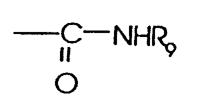
dans laquelle R₉ est hydrogène, alkyle linéaire ou ramifié en (C₁-C₁₀), glycosyle, 2-méthoxyéthyle, 2-diméthyamino-éthyle, (2,3 ou 4)-pyridyle ou (2,3 ou 4)-pyridyl-méthyle ; ou X et Y pris conjointement forment le cycle thiazolidinedione de la formule : 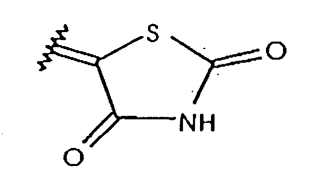 et les sels et esters pharmaceutiquement acceptables.

2. Composé de formule I comme défini dans la revendication 1 dans laquelle :
A, B, C, D, les lignes en pointillés, R et X sont comme défini dans la revendication 1 ;
Y est hydrogène et
Ar est une partie de la formule : 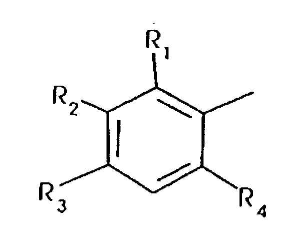 dans laquelle R₁, R₂, R₃ et R₄ sont hydrogène, alkyle en (C₁-C₃), alkoxy en (C₁-C₃) ou trifluorométhyle ;
et les sels et esters pharmaceutiquement acceptables.

3. Composé selon la formule I comme défini dans la revendication 1 dans laquelle :
A est CH ou CH₂ ;
B, C, D, R, X et les lignes en pointillés sont comme défini dans la revendication 1 ;
Ar est une partie de la formule : 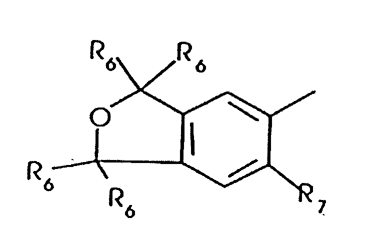
dans laquelle R₆ et R₇ sont comme défini dans la revendication 1 ; et
Y est hydrogène,
et les sels et esters pharmaceutiquement acceptables.

4. Composé selon la revendication 1 qui est l'acide E,E-3-méthyl-5-[2-(1,1,3,3-tétraméthyl-1,3-dihydro-5-isobenzofurannyl-1-cyclopent-l-yl] -2,4-pentadiénoïque.

5. Utilisation d'un composé selon l'une quelconque des revendications 1 à 4 dans la préparation d'un médicament pour le traitement ou la prévention de coronaropathie chez un mammifère.

6. Utilisation d'un composé selon l'une quelconque des revendications 1 à 4 dans la préparation d'un médicament pour l'augmentation des taux plasmatiques de HDL chez un mammifère.

7. Utilisation d'un composé selon l'une quelconque des revendications 1 à 4 dans la préparation d'un médicament pour la prévention de l'athérosclérose chez un mammifère.

8. Utilisation d'un composé selon l'une quelconque des revendications 1 à 4 dans la préparation d'un médicament pour traiter des cancers par induction de la différentiation des cellules tumorales chez un mammifère.

9. Composé de formule I comme défini dans l'une quelconque des revendications 1 à 4 pour l'utilisation comme produit pharmaceutique.

10. Procédé de préparation d'un composé de formule I comme défini dans la revendication 1 ou d'un sel ou ester pharmaceutiquement acceptable de celui-ci qui comprend une des étapes suivantes :
a) la réaction d'un composé de formule 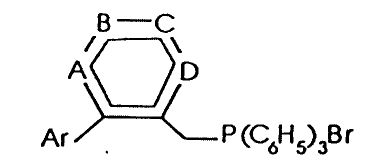 avec un aldéhyde de formule 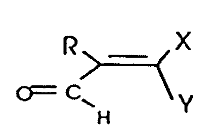 dans lesquelles Ar, A, B, C, D, R et les lignes en pointillés sont comme défini dans la revendication 1, X est CO₂R₈ et Y est hydrogène pour donner un composé correspondant de formule I ; ou
b) l'hydrolyse d'un composé de formule I où X est COR₈ pour donner un acide carboxylique où X est CO₂H ; ou
c) la réduction d'un composé de formule I où X est CO₂R₈ ou CO₂H pour donner un composé de formule I dans laquelle X est CH₂OH ; ou
d) la réaction d'un composé de formule I dans laquelle X est CO₂H avec un réactif d'activation et ensuite avec une amine de formule R₉NH₂ pour donner un amide de formule I dans laquelle X est CONHR₉ ; ou
e) la réaction d'un composé de formule VIII 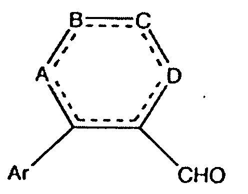 avec un ylide de formule 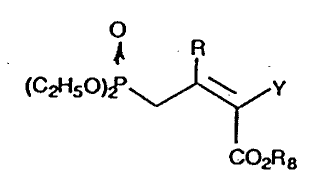 pour donner un composé correspondant de formule I dans laquelle Ar, A, B, C, D, R, Y et R₈ sont comme défini ci-dessus ; ou
f) l'oxydation d'un composé de formule I dans laquelle X est CH₂OH pour donner un composé de formule I dans laquelle X est CHO ; ou
g) la déshydratation d'un composé de formule I dans laquelle X est CONH₂ pour donner un composé de formule I dans laquelle X est CN, par exemple en utilisant P₂O₅ ; ou
h) la réaction d'un composé de formule I dans laquelle X est CN avec de l'azide de sodium pour donner un composé de formule I dans laquelle X est 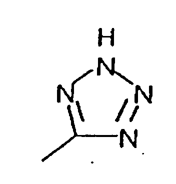 ou
i) la réaction d'un aldéhyde de formule 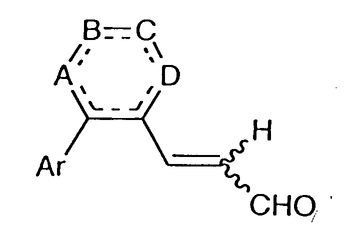 dans laquelle Ar, A, B, C, D et les lignes en pointillés sont comme défini ci-dessus avec la 2,4-thiazolidinedione pour donner un composé de formule I dans laquelle X et Y pris conjointement représentent

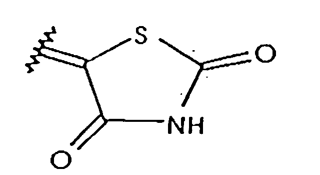

11. Procédé de préparation d'un composé de formule I selon la revendication 1, qui comprend la réaction d'un composé de formule ArBr ou ArI avec un alkyllithium, dans un solvant inerte, à -78°C à +30°C, puis l'addition de ZnCl₂ ; pour donner un composé de formule :
ArZnCl
la réaction de ArZnCl avec un composé de formule II 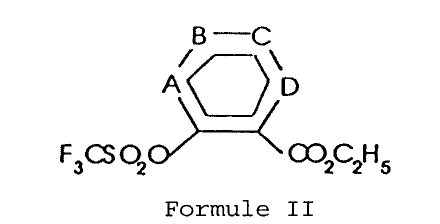 en présence d'un catalyseur de palladium(0), dans un solvant inerte, à 10°C à 60°C ; pour obtenir un composé de formule III : 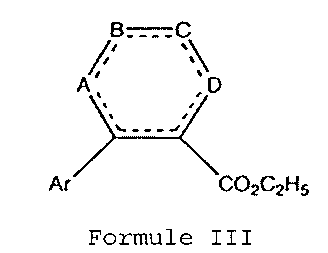 la réduction du composé de formule III avec un agent réducteur hydrure dans un solvant inerte à 0°C à 60°C ; pour donner un composé de formule IV : 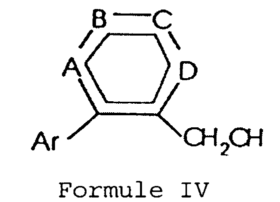 la réaction du composé de formule IV avec un bromhydrate de phosphine trisubstituée pour donner un composé de formule V : 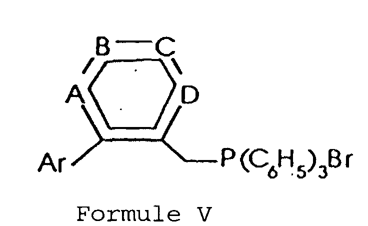 la réaction du composé de formule V avec une base, dans un solvant inerte à 0°C ; puis addition d'un aldéhyde de formule 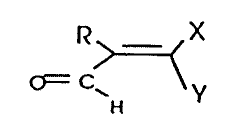 dans lequel R est comme défini dans la revendication 1 ; et X est CO₂R₈ et R₈ est comme défini dans la revendication 1 ; pour donner un composé de formule I.

12. Procédé de préparation d'un composé de formule I défini dans la revendication 1, dans lequel X est -CHO ; qui comprend la réaction d'un composé de formule I dans laquelle X est -CO₂H ou -CO₂R₈ et R₈ est comme défini dans la revendication 1 ; avec un agent réducteur hydrure, dans un solvant inerte, à 0°C à 60°C ; pour obtenir un composé de formule I dans laquelle X est -CH₂OH ; le traitement du composé de formule I dans laquelle X est CH₂OH avec un agent oxydant pour donner un composé de formule I dans laquelle X est -CHO.

13. Procédé de préparation d'un composé selon la revendication 1 dans lequel X est -C(O)-NHR₉ où R₉ est comme défini dans la revendication 1 ; qui comprend la réaction d'un composé de formule I dans laquelle X est -CO₂R₈ et R₈ est comme défini dans la revendication 1 : avec une base sous des conditions d'hydrolyse à 30°C à 100°C, puis l'acidification avec un acide minéral ; pour obtenir un composé de formule I dans laquelle X est -CO₂H ; la réaction du composé de formule I dans laquelle X est -CO₂H avec un réactif d'activation dans un solvant inerte, à 0°C à 25°C ; pour donner un intermédiaire ; l'addition d'une amine de formule R₉NH₂ à l'intermédiaire, où R₉ est comme défini dans la revendication 1 ; pour obtenir un composé de formule I.

14. Procédé de préparation d'un composé selon la revendication 1 dans lequel X est -CO₂R₈ et R₈ est comme défini dans la revendication 1 ;
qui comprend la réaction d'un composé de formule IV : 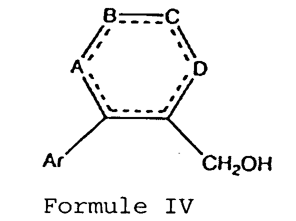 dans laquelle Ar et Y sont comme défini ci-dessus ; avec un agent oxydant, dans un solvant inerte à 0°C à 40°C ; pour donner un composé de formule VIII : 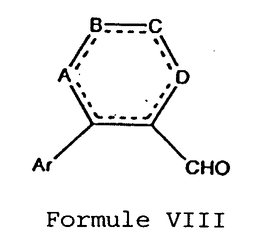 la réaction du composé de formule VIII avec un ylide de formule : 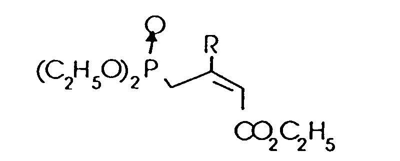 en présence d'une base dans un solvant inerte ; pour donner un composé de formule I.

15. Composition pharmaceutique qui comprend un composé selon l'une quelconque des revendications 1 à 5 et un support pharmaceutiquement acceptable.
